# EUROPÄISCHE PATENTSCHRIFT

(11) **EP 3 713 525 B1**
(45) Veröffentlichungstag und Bekanntmachung des Hinweises auf die Patenterteilung: **01.09.2021**
(21) Anmeldenummer: 18804605.6
(22) Anmeldetag: 16.11.2018
(51) Int. Cl.: A61F 5/01

(54) **HANDORTHESE SOWIE VERFAHREN ZUM ANPASSEN EINER HANDORTHESE**
HANDORTHOSIS AND PROCEDURE FOR ADAPTING A HANDORHTOSIS
ORTHESE DE LA MAIN ET PROCEDURE POUR ADAPTER UNE PROTHESE DE LA MAIN

(30) Priorität: 24.11.2017 DE 102017127890
(43) Veröffentlichungstag der Anmeldung: 30.09.2020
(73) Patentinhaber: Ottobock SE & Co. KGaA, 37115 Duderstadt (DE)
(72) Erfinder: FLOOD, Fredrik, 64135 Katrineholm (SE); KAHLMEYER, Guido, 37308 Siemerode (DE); BIALOWONS, Julia, 37083 Göttingen (DE); HARDT, Alexander, 35614 Asslar (DE); LIDOLT, Klaus, 37115 Duderstadt (DE)
(74) Vertreter: Gramm, Lins & Partner Patent- und Rechtsanwälte PartGmbB
(86) Internationale Anmeldenummer: PCT/EP2018/081629
(87) Internationale Veröffentlichungsnummer: WO 2019/101655

(56) Entgegenhaltungen:
- DE-A1-102006 041 441
- DE-U1-202004 005 876
- DE-U1-202015 008 342
- GB-A- 2 425 960

## Beschreibung

Die Erfindung betrifft ein Handstütztelement für eine Handorthese und eine Handorthese mit einer Manschette zum Befestigen der Handorthese an einem Handgelenk oder einem Unterarm eines Trägers der Handorthese, wenigstens einem Trägerelement und wenigstens einem Handstützelement, das an dem Trägerelement befestigbar ist. Die Erfindung betrifft zudem ein Verfahren zum Anpassen einer solchen Handorthese.

Handorthesen sind aus dem Stand der Technik seit langem bekannt. Sie verfügen über eine Manschette, mit der die Handorthese an dem Handgelenk oder dem Unterarm des Trägers angeordnet wird. Je nach Einsatzzweck der Handorthese ist diese Manschette unterschiedlich ausgebildet. So gibt es Manschetten, die sich nur über das Handgelenk und einen kleinen Teil des Unterarms erstrecken, um nur eine geringe Stützwirkung aufzubringen, wenn beispielsweise nur ein Daumen gestützt werden soll. Soll hingegen das Handgelenk an sich ruhig gestellt werden, ist eine größere Manschette notwendig, die auch starre oder nur wenig flexible Elemente, beispielsweise Schienen oder Stäbe, aufweisen kann.

Handorthesen können insbesondere verwendet werden, um mit einem Handstützelement die Handfläche und gegebenenfalls zusätzlich die Finger einer Hand zu stützen und beispielsweise Spastiken vorzubeugen. Ein solches Handstützelement wird dabei vorzugsweise auf der Handinnenseite, also volar angeordnet und die Hand liegt auf dem Handstützelement auf. Selbstverständlich ist es auch möglich, das Handstützelement auf der gegenüberliegenden Seite der Hand, also dorsal, anzuordnen, so dass die Hand an dem Handstützelement befestigt wird, um sie zu stützen.

Nachteilig ist, dass eine Vielzahl unterschiedlicher Handorthesen vorgehalten werden müssen. So müssen für unterschiedlich große Hände unterschiedlich große Handstützelemente vorgehalten werden. Ist das Handstützelement deutlich größer als die zu stützende Hand, ist es möglich, bei unbedachten Bewegungen der Hand mit dem Handstützelement an Gegenstände, beispielsweise Türen, Schränke oder Tische, anzustoßen. Dadurch werden Schläge und Erschütterungen auf die eigentlich zu stützende und gegebenenfalls ruhig zu stellende Hand übertragen, was unangenehm, gegebenenfalls schmerzhaft und therapeutisch nicht gewollt ist. Ist hingegen das Handstützelement deutlich kleiner als die zu stützende Hand, kann die benötigte Stützwirkung nicht in vollem Umfang erreicht werden, da beispielsweise die letzten Fingerglieder nicht mehr auf dem Handstützelement aufliegen können, so dass diese Fingerglieder nicht gestützt werden.

Zudem müssen für rechte und linke Hände unterschiedliche Handstützelemente vorgehalten werden.

Eine derartige Handorthese ist beispielsweise aus der DE 10 2006 041 441 A1, der DE 20 2004 005 876 U1 und der DE 20 2015 008 342 U1 bekannt. Eine Handstützeinrichtung wird in der GB 2 425 960 A beschrieben.

Hinzukommt, dass für unterschiedliche Grade an Bewegungsfreiheit, die durch das Handstützelement und die Handorthese weiterhin ermöglicht werden sollen, unterschiedliche Handstützelemente aus dem Stand der Technik bekannt sind. Soll die Hand im Wesentlichen bewegungsfrei bleiben, ist es ausreichend, ein Handstützelement vorzusehen, auf dem die Hand beispielsweise lose aufliegt. Das Handstützelement ist über die Manschette, mit der die Handorthese am Handgelenk oder dem Unterarm des Trägers angeordnet wird, unverlierbar an der Hand angeordnet, die Hand selbst muss am Handstützelement nicht befestigt werden.

Soll hingegen die Bewegungsfreiheit der Hand deutlich mehr eingeschränkt werden, um beispielsweise Spastiken vorzubeugen, kann es von Vorteil sein, die Hand insgesamt, jeden einzelnen Finger oder nur einzelne Finger am Handauflageelement zu befestigen. Da hierfür unterschiedliche Befestigungselemente, insbesondere Gurte, vorhanden sein müssen, müssen auch in diesen Fällen unterschiedliche Handorthesen mit unterschiedlichen Handstützelementen vorgehalten werden.

Dies ist nachteilig, da eine große Anzahl unterschiedlicher Handorthesen gefertigt und vorgehalten werden müssen, wodurch sich die Lagerhaltung verteuert.

Der Erfindung liegt daher die Aufgabe zugrunde, diese Nachteile zu beheben oder zumindest zu verringern.

Die Erfindung löst die gestellte Aufgabe durch das Handstützelement des Anspruchs 1, die sich dadurch auszeichnet, dass das Handstützelement eine Kontur aufweist, die symmetrisch zu einer Längsachse ausgebildet ist und auf beiden Seiten der Längsachse einen Daumenstützbereich aufweist. Die erfindungsgemäße dazugehörige Handorthese verfügt folglich über ein Handstützelement mit zwei Daumenstützbereichen. Auf diese Weise wird erreicht, dass das Handstützelement sowohl als Stützelement für eine rechte Hand als auch als Stützelement für eine linke Hand verwendet werden kann. Es ist daher nicht mehr notwendig, für rechte und linke Hände unterschiedliche Handstützelemente oder gar unterschiedliche Handorthesen vorzuhalten. Soll die Handorthese an eine rechte oder linke Hand angepasst werden, wird das nicht benötigte Daumenelement entfernt, beispielsweise mit einer Schere abgeschnitten. Selbstverständlich können auch andere Schneidwerkzeuge verwendet werden. Gegebenenfalls werden die Schnittkanten geschliffen und entgratet, um Verletzungsgefahren zu verringern und den Tragekomfort zu erhöhen. Vorteilhafterweise verfügt das Handstützelement über Markierungen, die stilisierte Konturen unterschiedlich großer rechter und/oder linker Hände darstellen. Das Handstützelement kann zum Anpassen der Handorthese an die Hand des Trägers der Handorthese auch in dieser Hinsicht zurecht geschnitten, gekürzt oder konfektioniert werden. Dazu wählt beispielsweise der Orthopädietechniker oder eine andere Person, die die Handorthese anpasst, eine passende Kontur einer rechten oder linken Hand aus und schneidet das Handstützelement entlang dieser Kontur zurecht. Auf diese Weise wird das Handstützelement sowohl an die zu versorgende Seite der Hand, also eine rechte Hand oder eine linke Hand, angepasst, als auch auf die Größe der jeweiligen Hand zugeschnitten. Auch in diesem Fall kann es sinnvoll sein, eventuell auftretende Schnittkanten zu entgraten oder zu glätten.

Vorzugsweise verfügen die Markierungen über Vertiefungen und/oder Durchbrüche. Dadurch wird das Schneiden entlang der Konturen und Markierungen vereinfacht.

Handelt es sich um Vertiefungen, verfügt das jeweilige Handstützelement bei diesen Markierungen über eine geringe Dicke, so dass ein Zerschneiden beispielsweise mit einer Schere, deutlich vereinfacht wird. Verfügen die Markierungen hingegen bereits über Durchbrüche, muss an diesen Stellen kein Schnitt mehr durchgeführt werden, sondern es müssen lediglich zwischen den einzelnen Durchbrüchen entlang der Markierung verlaufende Stege durchtrennt werden. Auch dadurch wird der Zuschnitt deutlich vereinfacht.

Vorzugsweise verfügt das Handstützelement über mehrere, vorzugsweise drei, vier oder fünf Durchbrüche, die sich parallel oder nahezu parallel zur Längsachse des Handstützelementes erstrecken. Diese Durchbrüche sind vorteilhafterweise so angeordnet, dass sie zwischen den Auflageflächen für die einzelnen Finger einer durch das Handstützelement zu stützenden Hand angeordnet sind. Durch sie können Gurte, Riemen oder andere Befestigungselemente hindurchgeführt werden, um auf diese Weise einzelne Finger, Fingergruppen oder die gesamte Hand an dem Handauflageelement zu befestigen. Die Gurte können aus einem elastischen oder einem inelastischen Material hergestellt sein und sind vorzugsweise mit Verschlusselementen, beispielsweise Schnallen, Klettverschlusselementen oder Druckknöpfen ausgerüstet, um eine Befestigung und Fixierung des jeweiligen Körperteils am Handstützelement gewährleisten zu können. Vorzugsweise sind die Befestigungselemente von dem Handstützelement zu entfernen, um sie beispielsweise durch andere Elemente zu ersetzen, falls beispielsweise die Länge der Befestigungselemente anzupassen ist, oder die Befestigungselemente oder das Handstützelement zu reinigen oder zu reparieren.

Vorzugsweise ist das Handstützelement aus einem Kunststoff hergestellt. Dadurch kann eine angenehme Haptik, ein geringes Gewicht und zudem eine leichte Durchtrennbarkeit des Handstützelementes insbesondere entlang der Markierungen erreicht werden. Zudem ist das Handstützelement vorzugsweise wasserunempfindlich.

In einer bevorzugten Ausgestaltung verfügt das Handstützelement auf der dem Trägerelement zugewandten Seite über wenigstens ein Befestigungselement, vorzugsweise ein Formschlusselement, das eingerichtet ist, mit einem am Trägerelement angeordneten korrespondierenden Befestigungselement oder Formschlusselement oder dem Trägerelement selbst zusammen zu wirken. Auf diese Weise ist es möglich, das Handstützelement an dem Trägerelement insbesondere formschlüssig zu befestigen. Handelt es sich beispielsweise um eine Clipsverbindung wird dadurch eine leicht lösbare Befestigung erreicht. Vorzugsweise ist das Handstützelement lösbar an dem Trägerelement befestigbar. Dadurch ist es nicht mehr notwendig, das Handstützelement bereits bei der Herstellung der Handorthese mit dem Trägerelement zu verbinden oder es an ihm zu befestigen. Vielmehr kann das Handstützelement beispielsweise vom Orthopädietechniker oder gegebenenfalls sogar vom Patienten an der gewünschten Stelle und in der gewünschten Orientierung und Position am Trägerelement angeordnet werden. Auch dadurch wird die Lagerhaltung verringert und die Flexibilität der Einsetzbarkeit erhöht.

Die Erfindung löst die gestellte Aufgabe zudem durch ein Handstützelement für eine hier beschriebene Handorthese. Die Erfindung löst die Aufgabe zudem durch ein Verfahren zum Anpassen einer Handorthese gemäß einem Ausführungsbeispiel der vorliegenden Erfindung, wobei bei dem Verfahren ein Daumenstützbereich des Handstützelementes entfernt, bevorzugt abgeschnitten wird. Vorteilhafterweise können dann die Schnittkanten geglättet, entgratet oder auf sonstige Weise nachbehandelt werden, um Verletzungsgefahren zu reduzieren und den Tragekomfort zu erhöhen. Vorteilhafterweise wird das Handstützelement entlang wenigstens einer der Markierungen zugeschnitten.

Mit Hilfe der beigefügten Zeichnungen wird nachfolgend ein Ausführungsbeispiel der vorliegenden Erfindung näher erläutert. Es zeigen
- Figuren 1 und 2 -: schematische Darstellungen eines Handstützelementes aus unterschiedlichen Perspektiven und
- Figuren 3 und 4 -: schematische Darstellungen eines Teils einer Handorthese.

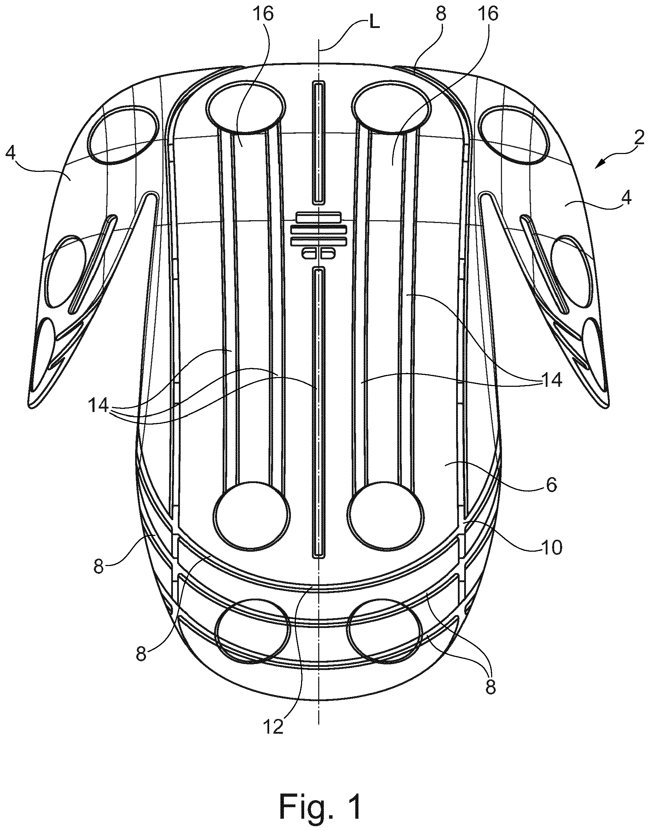

Figur 1 zeigt ein Handstützelement 2 für eine Handorthese gemäß einem ersten Ausführungsbeispiel der vorliegenden Erfindung. Das Handstützelement verfügt über zwei Daumenstützbereiche 4 und ist relativ zu seiner Längsachse L symmetrisch ausgebildet. Eine Handauflagefläche 6 verfügt über mehrere Markierungen 8, die in Form von Vertiefungen 10 und Durchbrüche 12 ausgebildet sind. Zum Zuschneiden des gezeigten Handstützelementes 2 wird eines der beiden Daumenstützelemente 4 entfernt. Soll das Handstützelement 2 für eine rechte Hand verwendet werden, wird der in Figur 1 links dargestellte Daumenstützbereich 4 entfernt. Wird umgekehrt das Handstützelement 2 für eine linke Hand verwendet, wird der in Figur 1 rechts dargestellte Daumenstützbereich 4 entfernt. Je nach Größe der Hand wird das Handstützelement 2 entlang einer der dargestellten Markierungen 8 zugeschnitten.

Zusätzlich verfügt das dargestellte Handstützelement 2 über Schlitze 14, durch die beispielsweise Gurte oder andere Befestigungselemente geführt am Handstützelemente befestigt werden können. Auf diese Weise können beispielsweise einzelne Finger einer Hand an einem Steg 16, der auf beiden Seiten durch jeweils einen Schlitz begrenzt wird, befestigt werden. Selbstverständlich ist es auch möglich, einen Gurt oder Riemen durch zwei nicht benachbarte Schlitze 14 zu führen und so mehrere Finger, alle Finger und/oder den Handteller an dem Handstützelement 2 zu befestigen.

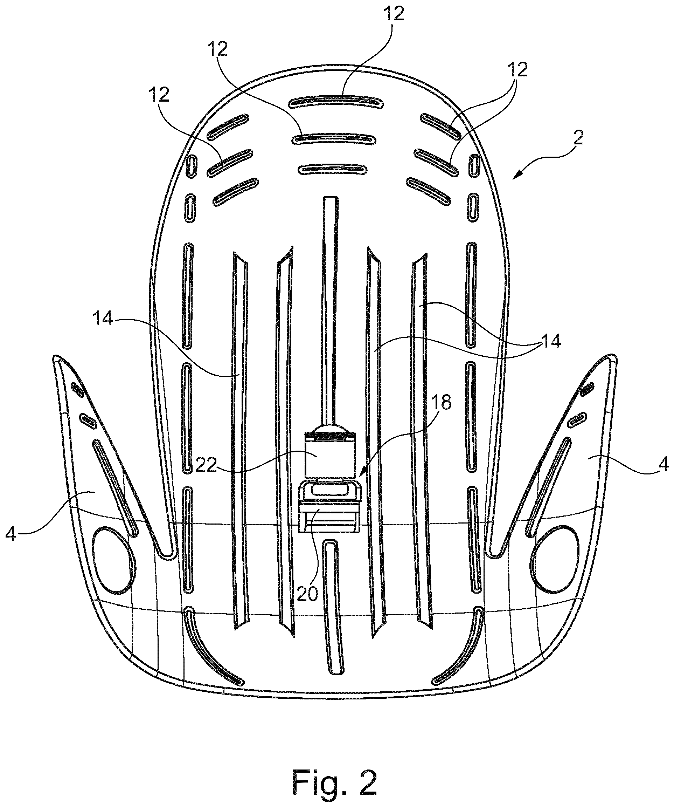

Figur 2 zeigt das Handstützelement 2 aus Figur 1 in einer anderen Perspektive. Gezeigt ist nun eine Darstellung von unten, während in Figur 1 die Darstellung von oben dargestellt ist. Die beiden Daumenstützbereiche 4, die Durchbrüche 12 und die Schlitze 14 sind auch von dieser Unterseite erkennbar. Zentral befindet sich ein Befestigungselement 18, das in diesem Fall ein erstes Formschlusselement 20 aufweist. Dieses ist in Form einer Rille ausgebildet und durch einen Verschluss 22, der schwenkbar angeordnet ist, verschließbar. Mit diesem Befestigungselement 18 kann das Handstützelement 2 an einem in den Figuren 1 und 2 nicht dargestellten Trägerelement befestigt werden.

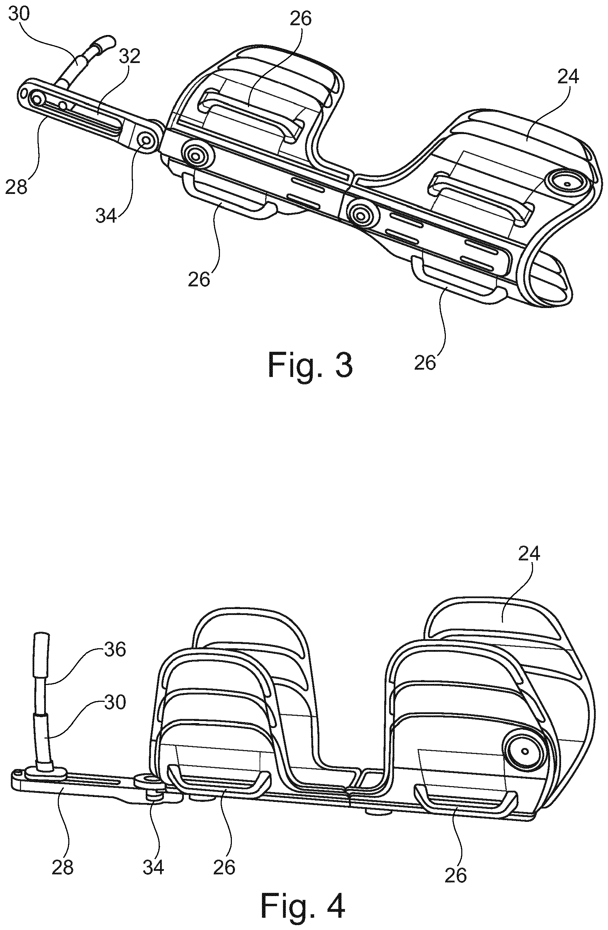

Die Figuren 3 und 4 zeigen weitere Bauteile einer Handorthese gemäß einem Ausführungsbeispiel der vorliegenden Erfindung. An einer beispielsweise aus einem Kunststoff hergestellten Unterarmschale 24 sind Ösen 26 angeordnet, durch die Gurte oder Riemen geführt werden können, womit die Unterarmschale 24 an einem Unterarm des Trägers angeordnet ist. In den Figuren 3 und 4 auf der linken Seite, die im angelegten Zustand einer Handorthese der Hand zuweist, befindet sich eine Stützstrebe 28, an der ein Trägerelement 30 angeordnet ist. Das Trägerelement 30 ist an einem Langloch 32 in der Stützstrebe 28 verschieblich angeordnet. Zudem kann es in zwei unterschiedlichen Orientierungen angeordnet werden. Man erkennt insbesondere in Figur 3, dass das Trägerelement 30 leicht gebogen ausgebildet ist. Während in den Figuren 3 und 4 ein Teil einer Handorthese für eine linke Hand ausgebildet ist, kann durch einfaches Umdrehen oder Umstecken des Trägerelementes 30 das Bauteil für eine Handorthese für eine rechte Hand passend gemacht werden.

Die Stützstrebe 28 ist über eine Winkeleinstellung 34 an der Unterarmschale 24 angeordnet, so dass auch hier ein Winkel eingestellt werden kann.

Insbesondere in Figur 4 ist zu erkennen, dass das Trägerelement 30 eine Verjüngung 36 aufweist, in der es einen reduzierten Durchmesser hat. Dies ist vorteilhaft, jedoch nicht zwangsläufig notwendig. Der Durchmesser und Querschnitt dieser Verjüngung 36 des Trägerelementes 30 ist an das erste Formschlusselement 20 am Handstützelement 2 angepasst, wie es beispielsweise in Figur 2 dargestellt ist. Auf diese Weise kann das Handstützelement 2 einfach und lösbar am Trägerelement 30 angeordnet werden.

### Bezugszeichenliste

- L: Längsachse
- 2: Handstützelement
- 4: Daumenstützbereich
- 6: Handauflagefläche
- 8: Markierung
- 10: Vertiefung
- 12: Durchbruch
- 14: Schlitz
- 16: Steg
- 18: Befestigungselement
- 20: erstes Formschlusselement
- 22: Verschluss
- 24: Unterarmschale
- 26: Öse
- 28: Stützstrebe
- 30: Trägerelement
- 32: Langloch
- 34: Winkeleinstellung
- 36: Verjüngung

## Patentansprüche

1. Handstützelement (2) für eine Handorthese, wobei das Handstützelement (2) an einem Trägerelement (30) der Handorthese befestigbar ist, **dadurch gekennzeichnet, dass** das Handstützelement (2) eine Kontur aufweist, die symmetrisch zu einer Längsachse (L) ausgebildet ist und auf beiden Seiten der Längsachse (L) einen Daumenstützbereich aufweist.

2. Handstützelement (2) nach Anspruch 1, **dadurch gekennzeichnet, dass** das Handstützelement (2) Markierungen (8) aufweist, die stilisierte Konturen unterschiedliche großer rechter und linker Hände darstellen.

3. Handstützelement (2) nach Anspruch 2, **dadurch gekennzeichnet, dass** die Markierungen (8) Vertiefungen (10) und/oder Durchbrüche (12) aufweisen.

4. Handstützelement (2) nach einem der vorstehenden Ansprüche, **dadurch gekennzeichnet, dass** das Handstützelement (2) aus einem Kunststoff hergestellt ist.

5. Handstützelement (2) nach einem der vorstehenden Ansprüche, **dadurch gekennzeichnet, dass** das Handstützelement (2) auf der dem Trägerelement (4) zugewandten Seite wenigstens ein Befestigungselement (18), vorzugsweise ein Formschlusselement (20) aufweist, das eingerichtet ist, mit einem am Trägerelement (30) angeordneten korrespondierenden Befestigungselement (36) oder Formschlusselement oder dem Trägerelement (30) zusammenzuwirken

6. Handorthese mit
a. einer Manschette zum Befestigen der Handorthese an einem Handgelenk oder einem Unterarm eines Trägers der Handorthese,
b. wenigstens einem Trägerelement (30) und
c. wenigstens einem Handstützelement (2) nach einem der vorstehenden Ansprüche.

7. Verfahren zum Anpassen einer Handorthese nach Anspruch 6, wobei bei dem Verfahren ein Daumenstützbereich (4) des Handstützelementes (2) entfernt, bevorzugt abgeschnitten wird.

8. Verfahren nach Anspruch 7, wobei das Handstützelement (2) entlang wenigstens einer der Markierungen (8) zugeschnitten wird.

## Claims

1. A hand support element (2) for a hand orthosis, wherein the hand support element (2) can be fixed to the carrier element (30),
**characterized by the fact that** the hand support element (2) features a contour that is designed to be symmetrical to a longitudinal axis (L) and comprises a thumb support area (4) on both sides of the longitudinal axis (L).

2. The hand support element (2) according to claim 1, **characterized by the fact that** the hand support element (2) features markings (8) which represent stylized contours of right and left hands of different sizes.

3. The hand support element (2) according to claim 2, **characterized by the fact that** markings (8) feature indentations (10) and/or openings (12).

4. The hand support element (2) according to one of the above claims, **characterized by the fact that** the hand support element (2) is made of a plastic.

5. The hand support element (2) according to one of the above claims, **characterized by the fact that** the hand support element (2) comprises at least one fixing element (18), preferably a positive-locking element (20), on the side that faces the carrier element (4), wherein said fixing element is configured to interact with a corresponding fixing element (36) or positive-locking element arranged on the carrier element (30) or with the carrier element (30) itself.

6. A hand orthosis with
a. a cuff for fixing the hand orthosis to a wrist joint or a lower arm of a wearer of the hand orthosis,
b. at least one carrier element (30) and
c. at least one hand support element (2) according to one of the above claims.

7. A method for adapting a hand orthosis according to claim 6, wherein the method comprises the removal, preferably the cutting off, of a thumb support area (4) of the hand support element (2).

8. The method according to claim 7, wherein the hand support element (2) is cut to size along at least one of the markings (8).

## Revendications

1. Élément de soutien de main (2) pour une orthèse de la main,
dans lequel
l'élément de soutien de main (2) peut être fixé à un élément porteur (30) de l'orthèse de la main,
**caractérisé en ce que**
l'élément de soutien de main (2) présente un contour qui est formé symétriquement par rapport à un axe longitudinal (L) et qui présente une zone de soutien de pouce de part et d'autre de l'axe longitudinal (L).

2. Élément de soutien de main (2) selon la revendication 1,
**caractérisé en ce que**
l'élément de soutien de main (2) présente des repères (8) représentant des contours stylisés de différentes tailles de la main droite et de la main gauche.

3. Élément de soutien de main (2) selon la revendication 2,
**caractérisé en ce que**
les repères (8) présentent des cavités (10) et/ou des traversées (12).

4. Élément de soutien de main (2) l'une des revendications précédentes,
**caractérisé en ce que**
l'élément de soutien de main (2) est réalisé en matière plastique.

5. Élément de soutien de main (2) selon l'une des revendications précédentes,
**caractérisé en ce que**
l'élément de soutien de main (2) présente, sur le côté tourné vers l'élément porteur (4), au moins un élément de fixation (18), de préférence un élément de coopération de forme (20), qui est conçu pour coopérer avec un élément de fixation (36) ou un élément de coopération de forme correspondant disposé sur l'élément porteur (30), ou avec l'élément porteur (30).

6. Orthèse de la main, comprenant
a. une manchette pour fixer l'orthèse de la main à un poignet ou à un avant-bras d'un porteur de l'orthèse de la main,
b. au moins un élément porteur (30), et
c. au moins un élément de soutien de main (2) selon l'une des revendications précédentes.

7. Procédé d'adaptation d'une orthèse de la main selon la revendication 6, dans lequel une zone de soutien de pouce (4) de l'élément de soutien de main (2) est retirée, de préférence coupée, dans le procédé.

8. Procédé selon la revendication 7,
dans lequel l'élément de soutien de main (2) est coupé le long de l'un au moins des repères (8).
